# EUROPEAN PATENT APPLICATION

(11) **EP 3 225 246 A1**
(43) Date of publication of application: **04.10.2017**
(21) Application number: 16163326.8
(22) Date of filing: 31.03.2016
(51) Int. Cl.: A61K 38/00, A61K 48/00, C07K 2/00, C07K 19/00, C12N 15/67

(54) **SYNTHETIC COMPOUND FOR IMPROVING EFFICIENCY OF TRANSFECTION**

(71) Applicant: Klinikum der Universität München, 80337 München (DE)
(72) Inventor: ROSENECKER, Joseph, 82541 Müsing (DE); GUAN, Shan, 80337 München (DE)
(74) Representative: Dr. Gassner & Partner mbB

(57) **Abstract**

The invention concerns a synthetic compound for improving efficiency of transfection of eukaryotic cells by means of an amphiphilic block copolymer which synthetic compound comprises a peptide having a targeting sequence and a nucleic acid binding sequence which nucleic acid binding sequence comprises or consists of at least four consecutive amino acid residues which are positively charged at pH 7.4, wherein the synthetic compound further comprises a hydrophobic moiety covalently linked to the peptide.

SEQUENCE LISTING

<110>  Klinikum der Universität München Großhadern

<120>  Synthetic compound for improving efficiency of transfection

<130>  569775EH

<160>  6

<170>  PatentIn version 3.5

<210>  1
<211>  17
<212>  PRT
<213>  Artificial Sequence

<220>
<223>  Synthetic Peptide

<220>
<221>  MOD_RES
<222>  (17)..(17)
<223>  AMIDATION

<400>  1

Val Lys Arg Lys Lys Lys Pro Lys Lys Arg Arg Arg Arg Lys Lys Trp
1               5                   10                  15

Lys

<210>  2
<211>  15
<212>  PRT
<213>  Artificial Sequence

<220>
<223>  Synthetic Peptide

<220>
<221>  MOD_RES
<222>  (15)..(15)
<223>  AMIDATION

<400>  2

Val Lys Arg Lys Lys Lys Pro Lys Lys Arg Arg Arg Arg Lys Lys
1               5                   10                  15

<210>  3
<211>  17
<212>  PRT
<213>  Artificial Sequence

<220>
<223>  Synthetic Peptide

<220>
<221>  MOD_RES
<222>  (17)..(17)
<223>  AMIDATION

<400>  3

Val Lys Arg Lys Thr Lys Pro Lys Lys Arg Arg Arg Arg Lys Lys Trp
1               5                   10                  15

Lys

<210>  4
<211>  26
<212>  PRT
<213>  Artificial Sequence

<220>
<223>  Synthetic Peptide

<400>  4

Trp Lys Lys Lys Lys Arg Arg Arg Arg Arg Lys Lys Lys Lys Gly Ala
1               5                   10                  15

Cys Ser Glu Arg Ser Met Asn Phe Cys Gly
            20                  25

<210>  5
<211>  26
<212>  PRT
<213>  Artificial Sequence

<220>
<223>  Synthetic Peptide

<400>  5

Trp Lys Lys Lys Lys Arg Arg Arg Arg Arg Lys Lys Lys Lys Gly Ala
1               5                   10                  15

Cys Tyr Gly Leu Pro His Lys Phe Cys Gly
            20                  25

<210>  6
<211>  25
<212>  PRT
<213>  Artificial Sequence

<220>
<223>  Synthetic Peptide

<400>  6

Trp Lys Lys Arg Arg Arg Arg Arg Arg Arg Arg Lys Lys Gly Ala Cys
1               5                   10                  15

Tyr Gly Leu Pro His Lys Phe Cys Gly
            20                  25

## Description

The present invention concerns a synthetic compound for improving efficiency of transfection of eukaryotic cells by means of an amphiphilic block copolymer, the synthetic compound for use in the treatment of a genetically caused disease of a human being or an animal by gene therapy, a use of the synthetic compound and a method for an *in vitro* transfection of eukaryotic cells in cell culture.

Gene transfer to the eukaryotic cells has many potential applications in inherited and acquired diseases, such as cystic fibrosis and cancer. At the heart of a successful gene therapy approach lies a gene delivery vector that is relevant to the pathology and compatible with the therapeutic schedule. Viral vectors are usually very efficient at promoting gene transfer into eukaryotic cells, but they are very immunogenic and not suitable for repeated dosing. Non-viral gene delivery vectors are much less efficient in terms of transfection efficiency but usually less likely induce a strong inflammatory response than viral gene delivery vectors. Thus non-viral gene delivery vectors may be relevant in pathologies that require repeated administrations. Among the current non-viral vectors, cationic lipids and cationic polymers are extensively studied and have clearly demonstrated their transfection efficiency *in vitro.* However, aggregation in tissue fluids, toxicity and low *in vivo* efficiency have thus far hampered their clinical use. Poloxamine-based block copolymers, comprising poly(ethylene oxide) and poly(propylene oxide) blocks, represent an attractive class of new non-viral gene delivery vectors.

US 2010/0179212 A1 discloses a pharmaceutical composition which combines a tetrafunctional copolymer with a nucleic acid. The copolymer is a poloxamine. The poloxamine may be in the form of one of the cationic mineral or organic salts thereof. The composition can be used to improve *in vivo* gene transfer.

From Evans, R. K., et al., Journal of Pharmaceutical Sciences, Vol. 93, No. 7, July 2004, pages 1924 to 1939 and from Hartikka, J., et al., J. Gene Med. 2008, 10, pages 770 to 782 plasmid DNA vaccine formulations containing the nonionic triblock copolymer adjuvant CRL1005 together or without the cationic surfactant benzalkonium chloride (BAK) are known.

From Zhang, J., et al., J. Gene Med. 2013, 15(0), pages 271 to 281 the use of the poloxamine Tetronic^{®} T904, a 4-arm polyethylene oxide / polypropylene oxide block copolymer for support of polycationic polymer/DNA complexes (polyplexes) based transfection is known. T904 significantly increased transfection efficiency of polyplexes based on 25 kDa branched polyethylenimine in a dose-dependent manner in the presence of serum in C6 glioma cells, human fibroblasts and mesenchymal stem cells.

Richard-Fiardo, P., et al., Biomaterials 2015 Mar, 45, pages 10 to 17 concerns the efficiency of the tetrafunctional block copolymer 704 as a non-viral gene delivery vector to the lungs of mice. The data obtained showed that the formulation 704 resulted in higher levels of reporter gene expression than the GL67A formulation currently being used in a clinical trial in cystic fibrosis patients. The inflammatory response associated with this gene transfer was lower than that induced by the GL67A formulation and the 704 formulation was amenable to repeated administrations.

From US 8,058,068 B2 transfection compositions in which a peptide is covalently linked to a transfection agent such as a lipid, cationic lipid or dendrimer are known. The peptide-lipid conjugate may be combined in a mixture of non-conjugated cationic and/or neutral lipids and then combined with the nucleic acid to form a peptide-lipid-nucleic acid lipid aggregate which facilitates introduction of the anionic nucleic acid through the cell membranes. Peptides useful in transfection compositions include functional portions that are fusagenic, function for nuclear or other sub-cellular localization, function for transport or trafficking or are receptor ligands. The methods disclosed in US 8,058 068 B2 involve contacting any cell, preferably a eukaryotic cell, with a transfection composition comprising a peptide including a fusagenic, membrane-permeabilizing, transport or trafficking sub-cellularlocalization, or receptor ligand peptide, optionally conjugated to a nucleic acidbinding group, or optionally conjugated to the transfection agent (lipid or dendrimer) wherein said peptide is non-covalently associated with the nucleic acid. In one embodiment, a peptide-nucleic acid complex (where the peptide can be conjugated to a nucleic acid binding group) is formed and then combined with a cationic lipid for transfection.

EP 1 294 908 B1 discloses a synthetic peptide consisting of an oligo-lysine-DNAbinding sequence and an SV40-nuclear localization sequence for improvement of transfection.

WO 2010/118213 A2 discloses peptides including a nucleic acid binding domain and a nuclear localization domain in order to form a peptide-nucleic acid delivery construct.

The problem to be solved by the present invention is to provide an improved synthetic compound for improving efficiency of transfection and for use in the treatment of a disease, a use of such a compound and an improved method for an *in vitro* transfection of eukaryotic cells in cell culture. Furthermore, a pharmaceutical composition shall be provided.

The problem of the present invention is solved by the subject-matter of claims 1, 8, 9, 12 and 14. Embodiments of the invention are subject-matter of claims 2 to 7, 10, 11, 13 and 15.

According to the invention a synthetic compound for improving efficiency of transfection of eukaryotic cells by means of an amphiphilic block copolymer is provided. The synthetic compound comprises a peptide having a targeting sequence and a nucleic acid binding sequence which nucleic acid binding sequence comprises or consists of at least of four, in particular at least five, in particular at least six, in particular at least seven, consecutive amino acid residues which are positively charged at pH 7.4. The synthetic compound further comprises a hydrophobic moiety covalently linked to the peptide.

The targeting sequence may be any sequence that causes a direction of the sequence to a position or a structure inside or on the surface of eukaryotic cells. The targeting sequence may be a ligand for a specific receptor on the surface of specific cells, such as a Fc receptor, or a sequence which specifically binds an antigene on the surface of specific cells. In particular the targeting sequence comprises or consists of a nuclear localization signal (NLS) sequence and/or a human airway epithelium ligand sequence. The targeting sequence allows it to specifically direct the nucleic acid to be transfected to the cells that should be transfected or to a specific compartment inside of cells in which compartment it shall exert its effect.

Even though poloxamine-based block copolymers are promising non-viral gene delivery vectors for *in vivo* application, the inventors of the present invention recognized that they are rather poor transfection agents in delivering nucleic acids into cultured cells. Data obtained from *in vitro* transfection study on airway epithelium cells revealed that transfection efficiency of complexes formed by *in vitro* transcribed (IVT) messenger RNA expressing Luciferase (MetLuc mRNA) and a broad range of concentrations of poloxamine 704 were all lower than 600 relative light units (RLU) per mg of cellular protein, which is similar to the intensity of background signal.

The inventors found that an improvement of the *in vitro* efficiency of transfection by means of an amphiphilic block copolymer also improves the *in vivo* efficiency of transfection by means of the amphiphilic block copolymer. They also found that the synthetic compound according to the invention drastically improved efficiency of transfection by means of an amphiphilic block copolymer. They further found that the synthetic compound, the nucleic acid to be transfected and the amphiphilic block copolymer form a ternary complex which ternary complex very efficiently effects transfection of the nucleic acid. The nucleic acid may be an RNA, in particular an mRNA or an siRNA, or a DNA and it may be present as a plasmid (pDNA or pRNA), a single strand or a double strand.

The inventors revealed that the highest transfection of MetLuc mRNA containing ternary complexes on cultured airway epithelium cells could reach around 400,000 RLU/mg cellular protein which is more than four times higher than that that could be reached with branched-polyethylenimine (brPEI, 25 kDa) based polymers which are considered as the "gold standard" in the art. In addition, the synthetic compound of the present invention also greatly supported poloxamine 704 mediated transfection of pDNA expressing Luciferase (MetLuc). After adding a synthetic compound according to the invention which compound contained a nuclear localization sequence (NLS), *in vitro* transfection efficiency of poloxamine 704/MetLuc pDNA significantly enhanced from 1,600 RLU/mg cellular protein to 1,700,000 RLU/mg cellular protein in airway epithelium cells after 3 days of incubation.

The effect of the nucleic acid binding sequence is that it causes a condensation of nucleic acids and facilitates an efficient cytoplasmic localization. The number and sequence of amino acid residues positively charged at pH 7.4 can vary. The positively charged amino acid residues may comprise or consist of histidine residue(s), arginine residue(s), lysine residue(s) and/or positively charged analog(s) of amino acid residue(s) and in particular only comprise or consist of arginine residue(s) and/or lysine residue(s). The amino acid residue(s) may be L-isomer(s) or D-isomer(s). The histidine residues(s) may be alpha-histidine or beta-histidine residue(s), the arginine residues(s) may be alpha-arginine or beta-arginine residue(s) and the lysine residues(s) may be alpha-lysine or beta-lysine residue(s). Different numbers and combinations of lysine residue(s), arginine residue(s), histidine residue(s), and positively charged analog(s) of amino acid residue(s) result in different condensing profiles and endosome/lysosome escaping ability. The positively charged analog of the amino acid residue may be an analog of an aspartic acid residue such as a residue of an N-substituteted aspartamide, d-aspartamide, or beta-aspartamide, or an analog of a glutamatic acid residue such as a residue of an N-substituteted glutamide, d-glutamide, or beta-glutamide. "N-substituteted" means that the carboxyl group on the side chain of aspartic acid residue or glutamic acid residue is modified with a chemical group containing primary, secondary and/or tertiary amine(s). For example, an N-substituteted aspartamide may be 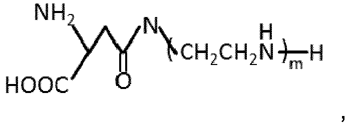 wherein m ≥ 1 and an N-substituteted glutamide may be 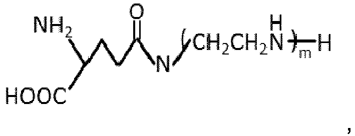 wherein m ≥ 1.

The hydrophobic moiety can be covalently linked to any part of the peptide. In one embodiment of the invention the hydrophobic moiety is covalently linked to the amino terminus or to the carboxy terminus of the peptide. The hydrophobic moiety may comprise or consist of lipoic acid, lipoamide, a tetradecyl residue, a cholesteryl residue, or a peptide having a sequence with more than 50% amino acid residues with hydrophobic side chains, in particular amino acid residues of phenylalanine, tyrosine and/or tryptophan. An advantage of lipoic acid is that it is produced naturally in the human body and commonly used as an antioxidant drug for treating diseases such as diabetes and HIV.

Hydrophobicity and therewith efficiency of transfection can further be increased by amidation of the carboxy terminus of the peptide such that the C-terminus of the peptide is formed by a -CONH₂ moiety.

The lipoic acid and the lipoamide can firmly interact with the hydrophobic part of the amphiphilic block copolymer. The disulfide bond localized in the dithiolane of lipoic acid and lipoamide could be specifically cleaved by glutathione (GSH) which exists in high level in the cytoplasm of eukaryotic cells. As a result hydrophobicity of the lipoic acid or the lipoamide is decreased and its interaction with the amphiphilic block copolymer is weakened. This is followed by release of the synthetic compound according to the invention from the amphiphilic block copolymer and an enhanced release of the nucleic acid from the delivery system. As a result a higher transfection rate is achieved.

The peptide may consist of the targeting sequence and the nucleic acid binding sequence. The peptide may comprise or consist of amino acid sequence SEQ ID NO: 1, SEQ ID NO: 2, SEQ ID NO: 4, SEQ ID NO: 5, or SEQ ID NO: 6.

The maximal length of the peptide may be 30, in particular 26, in particular 20, in particular 17, in particular 15, in particular 12, in particular 11, amino acid residues. The shorter the peptide is the lesser is the probability that it exerts an antigenic effect. This may be particular important if repeated administration is intended.

The synthetic compound may consist of the peptide and the hydrophobic moiety.

The invention also concerns the synthetic compound according to the invention for use in the treatment of a genetically caused disease of a human being or an animal by gene therapy, wherein the synthetic compound is used together with the amphiphilic block copolymer and a nucleic acid to be transferred in cells of the human being or the animal. The synthetic compound can be present in a nebulized form. The animal may be a mammal. The genetically caused disease may be cystic fibrosis or a cancer.

The invention also concerns a pharmaceutical composition comprising any synthetic compound according to the invention together with a pharmaceutically acceptable carrier. The pharmaceutically acceptable carrier may comprise or consist of water or a salt solution, in particular a physiologic salt solution, Tyrode's solution, an Eagle's Minimum Essential Medium such as Opti-MEM^{®} (purchased from ThermoFisher Scientific), a sodium chloride solution or a calcium chloride solution. The pharmaceutical composition may also be prepared as a dry powder formulation. In this case the pharmaceutically acceptable carrier can comprise or consist of an albumin, a polyethylene glycol (PEG), or a saccharide such as trehalose, mannose or mannitol. The pharmaceutical composition may also comprise the previously described nucleic acid and the previously described amphiphilic block copolymer. The pharmaceutical composition may be for use in the treatment of a genetically caused disease of a human being or an animal by gene therapy.

The invention further concerns the use of the synthetic compound according to the invention together with an amphiphilic block copolymer and a nucleic acid to be transferred in eukaryotic cells for an *in vitro* transfection of the eukaryotic cells in cell culture.

The invention also concerns a method for an *in vitro* transfection of eukaryotic cells in cell culture comprising the following steps:
a1) Mixing a plurality of molecules of the synthetic compound according to the invention with a plurality of molecules of an amphiphilic block copolymer to allow the formation of binary complexes,
b1) mixing the formed complexes with a plurality of molecules of a nucleic acid to be transferred into the cells to allow the formation of ternary complexes,
   or
a2) mixing a plurality of molecules of a nucleic acid to be transferred into the cells with a plurality of molecules of an amphiphilic block copolymer to allow the formation of binary complexes,
b2) mixing the formed complexes with a plurality of molecules of the synthetic compound according to the invention to allow the formation of ternary complexes
   and
c) contacting the ternary complexes with the cells to be transfected.

In each case of the synthetic compound for use in the treatment of a genetically caused disease, the use of the synthetic compound and the method for the *in vitro* transfection, the amphiphilic block copolymer may be a poloxamer, in particular a poloxamine, in particular an ethylene oxide - propylene oxide copolymer with tetraether with (1,2-ethanedinitrilo)tetrakis(propanol), in particular poloxamine 304, 704 or 904.

The invention is further illustrated on basis of the following embodiments.
- Fig. 1a to 1d: show *in vitro* transfection profiles of poloxamine 704 (P704) in human bronchial cells (BEAS-2B and 16HBE cells).
- Fig. 2a to 2d: show transfection efficiencies of mRNA containing ternary complexes in human bronchial epithelial cells depending on the concentrations of P704 and synthetic compound 0 (DL).
- Fig. 3a to 3d: show transfection efficiencies of pDNA containing ternary complexes in human bronchial epithelial cells depending on the concentrations of P704 and synthetic compound 0 (DL).
- Fig. 4a to 4c: show the *in vitro* mRNA transfection profiles of the ternary complexes depending on the concentration of mRNA in the ternary complexes, on different media used in complex preparation and on different methods of complex preparation.
- Fig. 5a to 5d: show comparisons of *in vitro* transfection efficiencies of P704 based ternary complexes with that of other non-viral vectors.
- Fig. 6a and 6b: show comparisons of toxicities of P704 based ternary complexes, Lipofectamine^{®} 2000 based formulations and brPEI based formulations.
- Fig. 7a and 7b: show transfection efficiencies of P704 based ternary complexes and Lipofectamine^{®} 2000 based formulations before and after nebulization.
- Fig. 8a and 8b: show transfection efficiencies of pDNA containing P704 based ternary complexes comprising different synthetic compositions according to the invention.
- Fig. 9a and 9b: show comparisons of maximum *in vitro* transfection efficiencies of pDNA containing P704 based ternary complexes comprising different synthetic compositions.
- Fig. 10a and 10b: show transfection efficiencies of mRNA containing P704 based ternary complexes comprising different synthetic compositions according to the invention.
- Fig. 11a and 11b: show comparisons of maximum *in vitro* transfection efficiencies of mRNA containing P704 based ternary complexes comprising different synthetic compositions.

The following synthetic compositions were synthesized:
1. Compound 0 (DL): NH₂-VKRKKKPKKRRRRKKWK(DL-lipoic acid)-CONH₂ (peptide sequence of this composition: SEQ ID NO: 1),
2. compound 0 (R): NH₂-VKRKKKPKKRRRRKKWK(R-lipoic acid)-CONH₂ (peptide sequence of this composition: SEQ ID NO: 1),
3. compound 1: NH₂-VKRKKKPKKRRRRKK-CONH₂ (peptide sequence SEQ ID NO: 2),
4. compound 3: NH₂-VKRKTKPKKRRRRKKWK(DL-lipoic acid)-CONH₂ (peptide sequence SEQ ID NO: 3),
5. compound 4: DL-lipoic acid-WKKKKRRRRRKKKK-GACSERSMNFCG (peptide sequence SEQ ID NO: 4),
6. compound 5: DL-lipoic acid-WKKKKRRRRRKKKK-GACYGLPHKFCG (peptide sequence SEQ ID NO: 5) and
7. compound 7: DL-lipoic acid-WKKRRRRRRRRKK-GACYGLPHKFCG (peptide sequence SEQ ID NO: 6).

In compounds 0 and 3 the hydrophobic moiety in form of the lipoic acid is in each case covalently bound to the peptide via the side chain of the C-terminal lysine moiety. In compounds 4, 5 and 7 the hydrophobic moiety in form of the lipoic acid is in each case covalently bound to the N-terminus of the peptides according to SEQ ID NO: 4, SEQ ID NO: 5 and SEQ ID NO: 6.

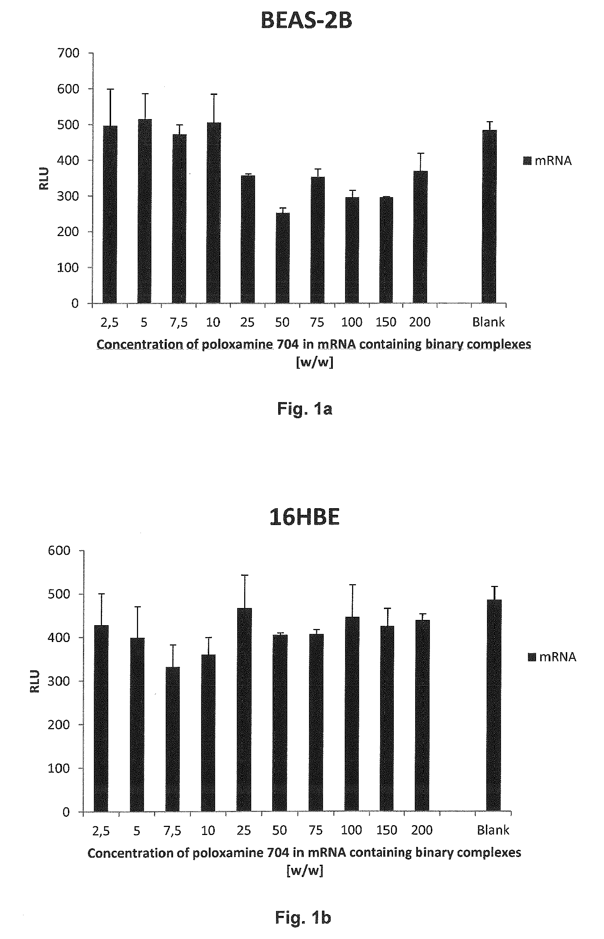

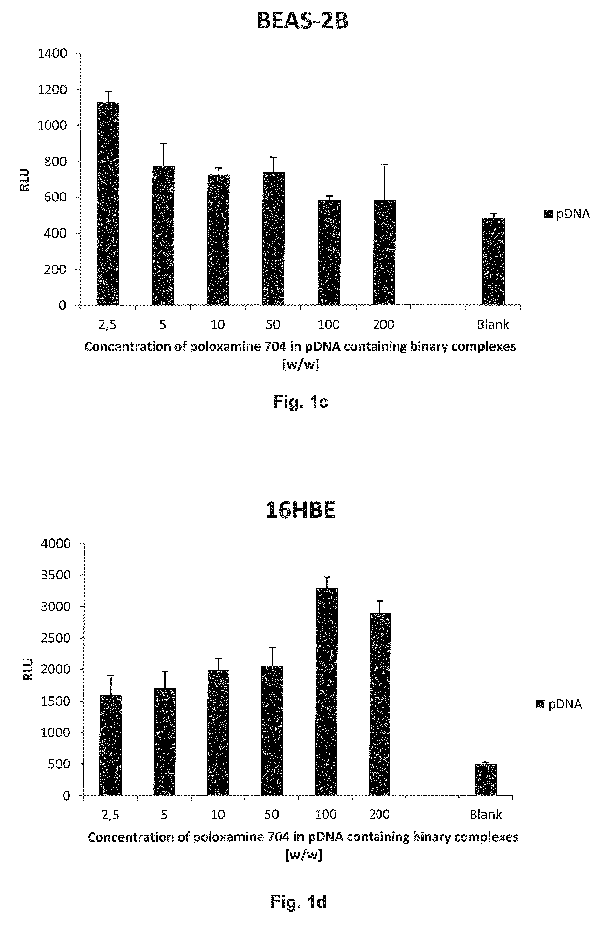

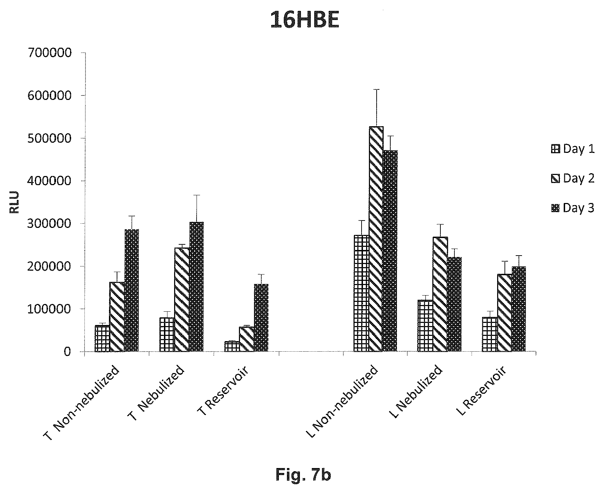

The only difference between compound 0 (DL) and compound 0 (R) is that the lipoic acid is DL-lipoic acid in case of compound 0 (DL) and R-lipoic acid in case of compound 0 (R). DL-lipoic acid and R-lipoic acid are enantiomers. Transfection efficiencies of ternary complexes formed by P704, a nucleic acid and compound 0 (DL) or compound 0 (R) are similar. The following experiments were performed by use of compound 0 (DL). Results are shown in Fig. 1 a to Fig. 7b.

MetLuc mRNA was complexed with P704 at different concentrations from 2.5 to 200 (w/w, relative to nucleic acids) in Tyrode solution. After 30 min incubation at room temperature (RT), samples containing 400 ng mRNA or pDNA were added in each well of a 96-well plate containing pre-seeded cells and incubated with cells in opti-MEM (without serum and antibiotics) for 4 h at 37 °C in a humidified atmosphere (95% air, 5% CO₂). Luciferase activity in 50 µl supernatants from transfected BEAS-2B cells (Fig. 1 a) or 16HBE cells (Fig. 1 b) was assayed after 24 h and its activity is expressed in relative light units (RLU). MetLuc pDNA/P704 binary complexes were prepared in the same way as described above. Luciferase activity in transfected BEAS-2B cells (Fig. 1 c) or 16HBE cells (Fig. 1 d) was measured 48 h after transfection and its activity is expressed in RLU. The data are given as mean ± standard deviation (SD) (n>5) (RLU = Relative Light Units) in Figs. 1 a to 1d.

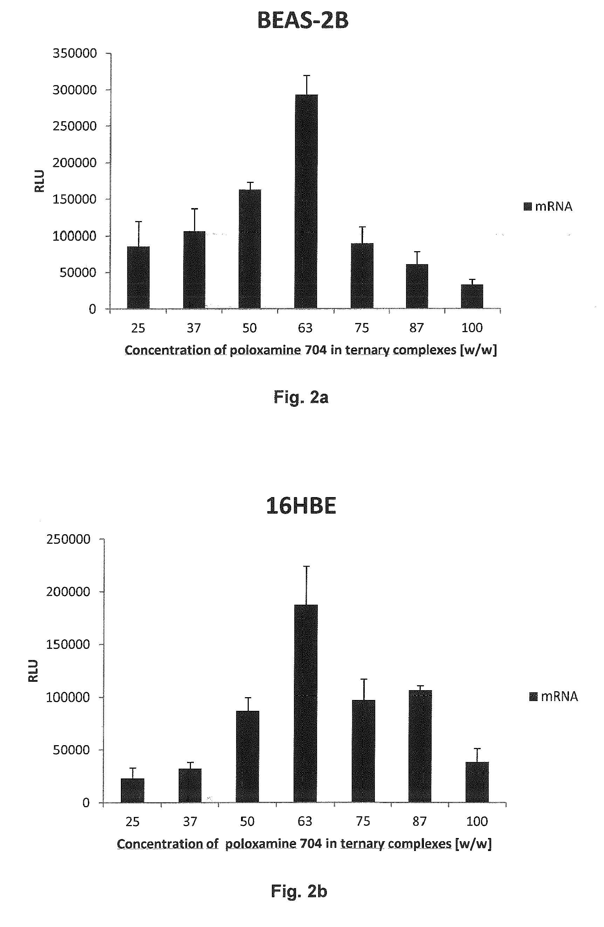

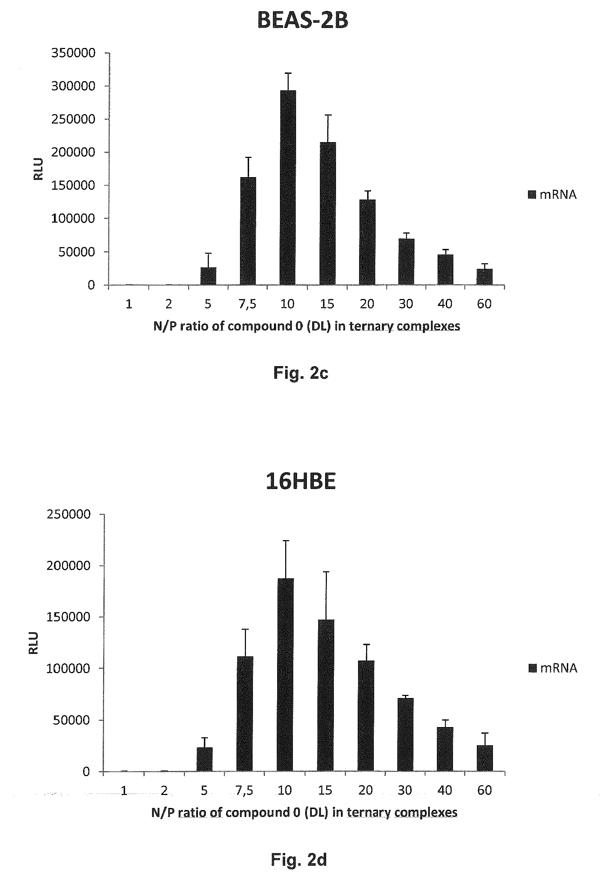

In order to investigate the influence of P704 concentration on the transfection of mRNA containing ternary complexes, synthetic compound 0 (DL) was first mixed at charge ratio (Negative-to-Positive (N/P) ratio) of 10 with equal volume of P704 at different concentrations from 25 to 100 (w/w, relative to nucleic acids). After 20 min incubation at room temperature (RT), equal amounts of MetLuc mRNA (400 ng/well) were added into above-mentioned solutions and mixed thoroughly. The final solutions were added to pre-seeded BEAS-2B cells (Fig. 2a) and 16HBE cells (Fig. 2b) after 20 min incubation at RT and incubated for 4 h at 37 °C in a humidified atmosphere. To evaluate the effect of concentration of synthetic compound 0 (DL) on the transfection of mRNA containing ternary complexes, P704 was first mixed at a concentration of 63 (w/w, relative to nucleic acids) with equal volume of synthetic compound 0 (DL) at different charge ratios (N/P ratios) from 1 to 60 and incubated at RT for 20 min. Then each of the resulting solutions were mixed with MetLuc mRNA (400 ng/well) and incubated for another 20 min. Samples were added to pre-seeded BEAS-2B cells (Fig. 2c) and 16HBE cells (Fig. 2d), and incubated for 4 h at 37 °C in a humidified atmosphere. Luciferase activity was measured as mentioned above. The results are given as mean ± SD of three independent experiments in Figs. 2a to 2d.

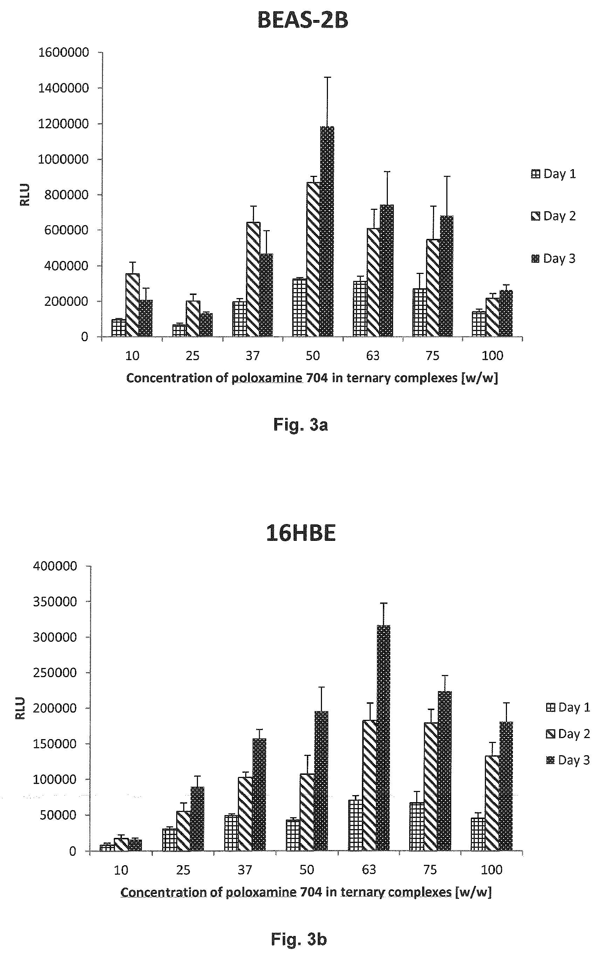

In order to investigate the effect of P704 concentration on the transfection rate of pDNA containing ternary complexes, synthetic compound 0 (DL) was first mixed at N/P ratio of 30 (for BEAS-2B cell transfection) or 20 (for 16HBE cell transfection) with equal volume of P704 at different concentrations from 10 to 100 (w/w, relative to nucleic acids). After 20 min incubation at RT, equal amounts of MetLuc pDNA (400 ng/well) were added into each of the above-mentioned solutions and mixed thoroughly. The final solutions were given to pre-seeded BEAS-2B cells (Fig. 3a) and 16HBE cells (Fig. 3b) after 20 min incubation at RT and incubated for 4 h at 37 °C in a humidified atmosphere. Luciferase activity was measured on day 1, day 2 and day 3 after transfection using the same method as mentioned above. The results are given as mean ± SD of three independent experiments in Figs. 3a and 3b.

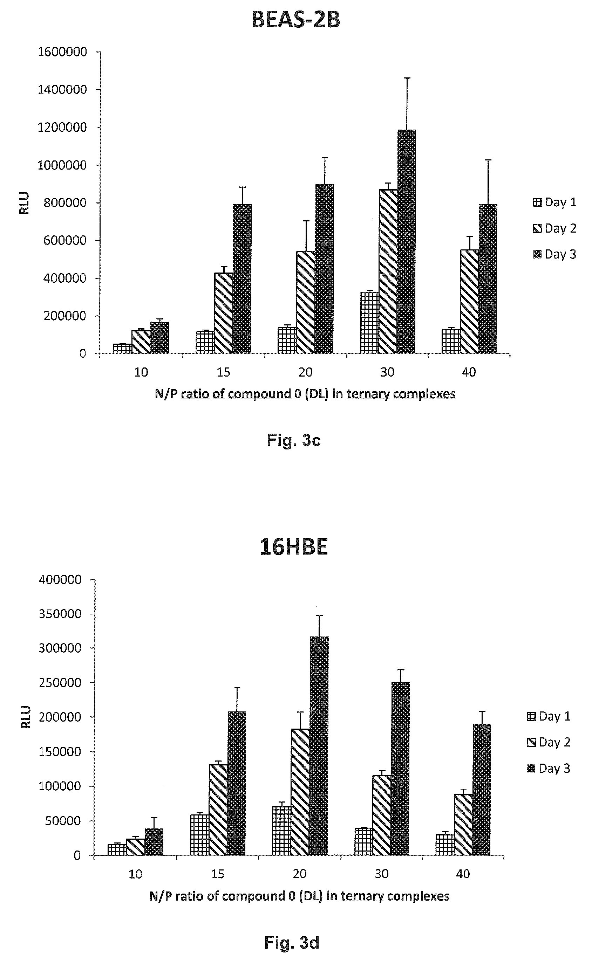

To evaluate the influence of concentration of synthetic compound 0 (DL) on the transfection of pDNA containing ternary complexes, P704 at concentrations of 50 (w/w, relative to nucleic acids) for BEAS-2B cell transfection or 63 (w/w, relative to nucleic acids) for 16HBE cell transfection was first mixed with equal volume of synthetic compound 0 (DL) at different N/P ratios in a range of 10 to 40 and incubated at RT for 20 min. Each of the resulting solutions was mixed with MetLuc pDNA (400 ng/well) and incubated for another 20 min. Samples were added to pre-seeded BEAS-2B cells (Fig. 3c) and 16HBE cells (Fig. 3d), and incubated for 4 h at 37 °C in a humidified atmosphere. Luciferase activity was measured on day 1, day 2 and day 3 after transfection using the same method as mentioned above. The results are given as mean ± SD of three independent experiments in Figs. 3c and 3d.

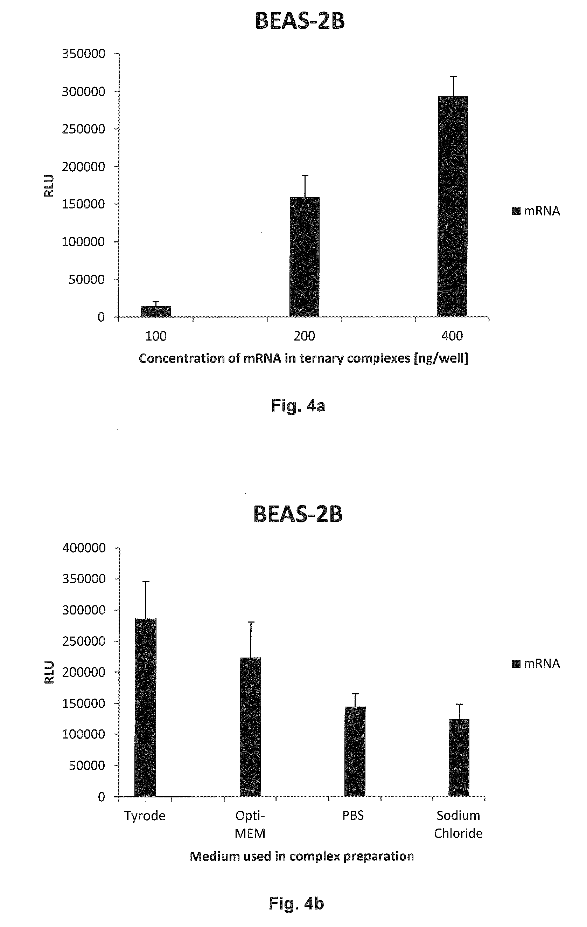

MetLuc mRNA was used at concentrations of 100, 200 or 400 ng/well to form ternary complexes with P704 at concentration of 63 (w/w, relative to nucleic acids) and synthetic compound 0 (DL) at N/P ratio of 10 using same method as described above. Luciferase activities were measured 24 h after transfection. The results are shown in Fig. 4a.

The transfection efficiencies of ternary complexes formed in Tyrode solution, opti-MEM, PBS and sodium chloride with MetLuc mRNA at concentration of 400 ng/well, P704 at concentration of 63 (w/w, relative to nucleic acids) and compound 0 (DL) at N/P ratio of 10 were evaluated 24 h after transfection. The results are shown in Fig. 4b.

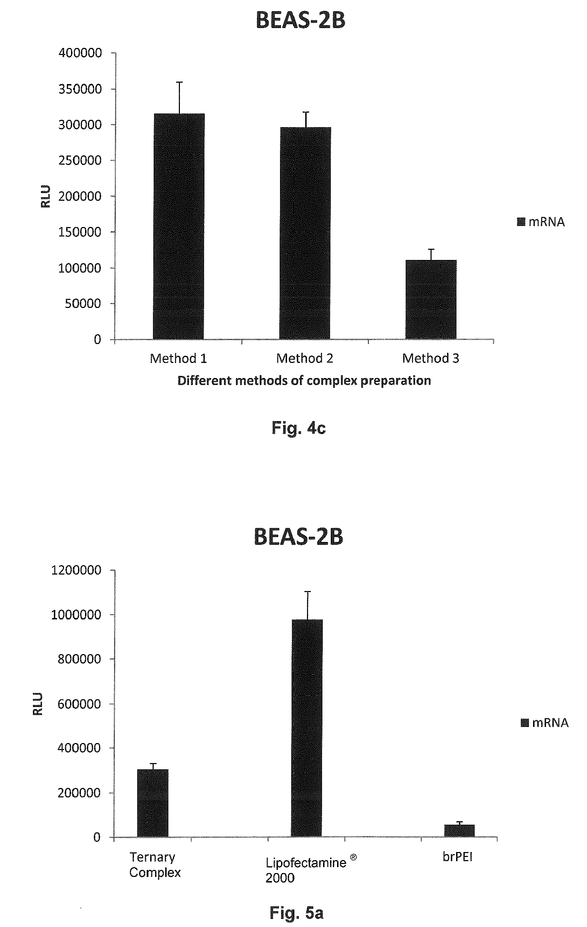

mRNA containing ternary complexes were prepared in three different ways, and their transfection rates were evaluated 24 h after transfection. Method 1: Equal amounts of P704 at concentration of 63 (w/w, relative to nucleic acids) and synthetic compound 0 (DL) at N/P ratio of 10 were firstly mixed and incubated for 20 min at RT, then equal volume of mRNA solution (400 ng/well) was added into the above solution in a second step and incubated for another 20 min at RT. Method 2: P704 and mRNA solution were mixed and incubated for 20 min at RT, followed by addition of equal volumes of synthetic compound 0 (DL) and incubation for another 20 min at RT. Method 3: Synthetic compound 0 (DL) was complexed with mRNA by 20 min incubation at RT, then equal volume of P704 solution was added and incubated for another 20 min to form ternary complexes. The concentrations of all the ingredients used in these methods were the same. The data shown in Fig. 4c are given as mean ± SD (n>5).

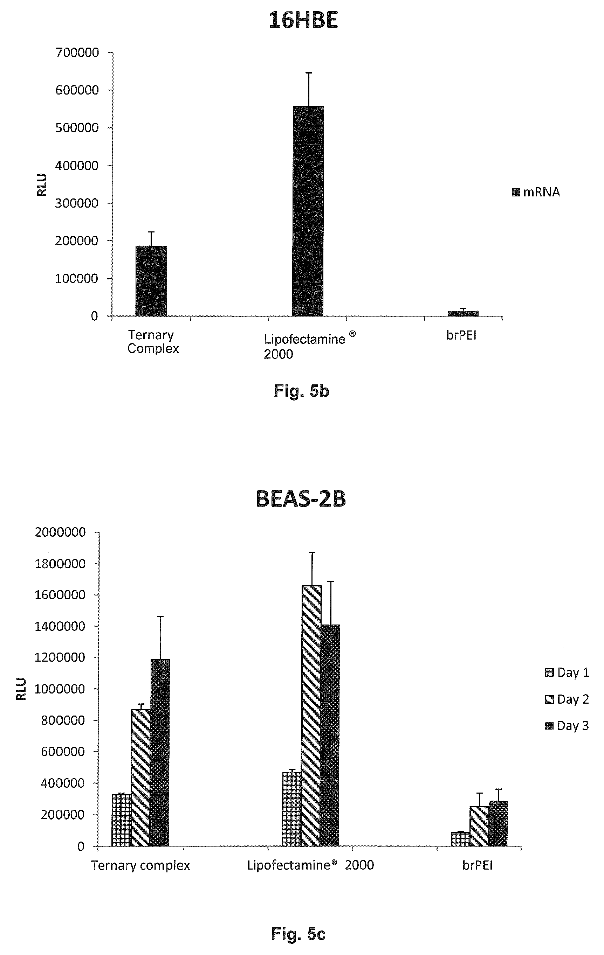

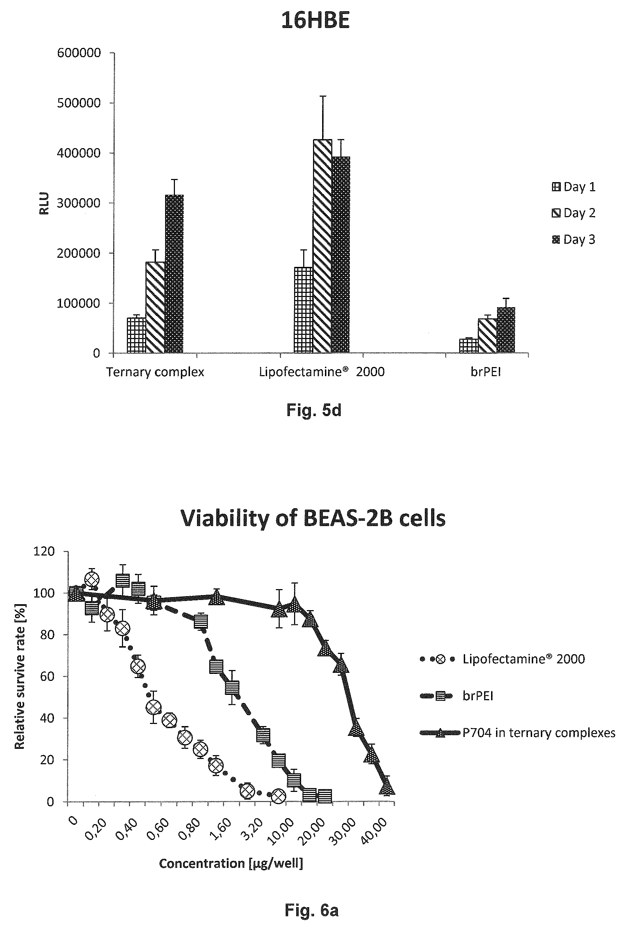

Luciferase activity (24 h after transfection) of MetLuc mRNA containing ternary complexes was compared to *in vitro* transfection agent Lipofectamine^{®} 2000 and 25 kDa branched polyethylenimine (brPEI) in BEAS-2B cells (Fig. 5a) and 16HBE cells (Fig. 5b). Lipofectamine^{®} 2000 is commercially available from Fisher Scientific GmbH, 58239 Schwerte, Germany. Luciferase activities obtained with MetLuc pDNA containing ternary complexes were measured in BEAS-2B cells (Fig. 5c) and 16HBE cells (Fig. 5d) on day 1, day 2 and day 3 after transfection and compared to those of Lipofectamine^{®} 2000 and brPEI. P704 at concentration of 63 (w/w, relative to nucleic acids), compound 0 (DL) at N/P ratio of 10 and mRNA at concentration of 400 ng/well were used to form ternary complexes for transfection of both BEAS-2B and 16HBE cells. P704 at concentration of 50 (w/w, relative to nucleic acids), compound 0 (DL) at N/P ratio of 30 and pDNA at concentration of 400 ng/well were used to form ternary complexes for transfection of BEAS-2B cells and P704 at concentration of 63 (w/w, relative to nucleic acids), compound 0 (DL) at N/P ratio of 20 and pDNA at concentration of 400 ng/well were used to form ternary complexes for transfection of 16HBE cells. Complexes composed of Lipofectamine^{®} 2000 at concentration of 0.4 µl/well (for BEAS-2B cell transfection) or 0.5 µl/well (for 16HBE cell transfection) and 400 ng/well mRNA or pDNA were prepared according to the manufacturer's instructions and used as positive lipoplex (lipid based non-viral vector) control. Complexes composed of brPEI at N/P ratio of 20 and 400 ng/well mRNA or pDNA were formed in injectable water (Ampuwa^{®}) and served as positive polyplex (polymer based non-viral vector) control. The results are given as mean ± SD of three independent experiments in Figs. 5a to 5d.

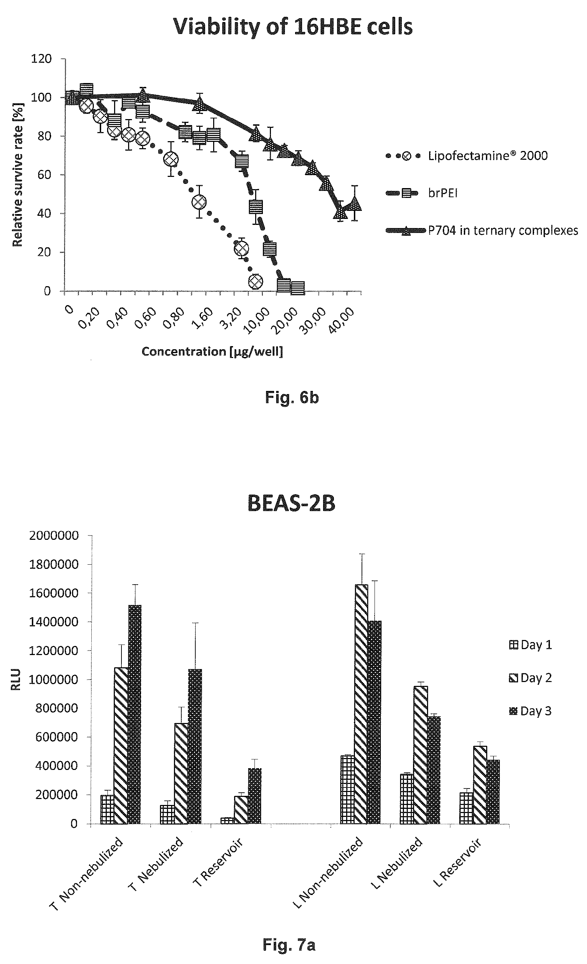

P704 based ternary complexes were prepared by mixing MetLuc pDNA (400 ng/well) with pre-mixed solutions of P704 at indicated concentrations ranging from 0.5 to 40 µg/well and synthetic compound 0 (DL) at N/P ratio of 30 in Tyrode solution. Lipofectamine^{®} 2000 in concentration ranges of 0.1 to 5 µg/well was complexed with 400 ng/well MetLuc pDNA in opti-MEM. brPEI based formulations were obtained by incubation of brPEI at different concentrations ranging from 0.1 to 20 µg/well and 400 ng/well MetLuc pDNA in injectable water for 30 min at RT. BEAS-2B cells (Fig. 6a) and 16HBE cells (Fig. 6b) were incubated with above-mentioned samples for 4 h at 37 °C in a humidified atmosphere, then fresh media containing 10% heat-inactivated fetal bovine serum and 100 U/ml penicillin and 100 µg/ml streptomycin were added to substitute pDNA containing samples. An MTT assay was performed 24 h post transfection. Non-treated cells served as positive control. The data are given as mean ± SD (n>5) in Figs. 6a and 6b.

P704 based ternary complexes for BEAS-2B cell transfection comprised P704 at concentration of 50 (w/w, relative to nucleic acids) and synthetic compound 0 (DL) at N/P ratio of 30. P704 based ternary complexes for 16HBE cell transfection comprised P704 at concentration of 63 (w/w, relative to nucleic acids) and synthetic compound 0 (DL) at N/P ratio of 20. Both ternary complexes comprised 400 ng/well MetLuc pDNA and were prepared in Tyrode solution. Lipofectamine^{®} 2000 at concentrations of 0.4 µl/well (for BEAS-2B cell transfection) and 0.5 µl/well (for 16HBE cell transfection) were mixed with 400 ng/well MetLuc pDNA in opti-MEM, respectively. A fraction of complexes was kept apart and used as a "non-nebulized" control. The rest of the solutions was aerosolized for 5 minutes by employing PARI Boy^{®} Nebulizer. The nebulized solutions were collected in a separate tube. Non-nebulized control, Collected solution (nebulized) and solution remaining in the nebulizer (reservoir) were incubated with BEAS-2B cells (Fig. 7a) and 16HBE cells (Fig. 7b) for 4 h at 37 °C in a humidified atmosphere. Luciferase activity in 50 µl of supernatants was assayed 24 h, 48 h, and 72 h after transfection. The luciferase activity is expressed in RLU. The cell culture media was replaced each day after sampling. In the figures "T" means "P704 based ternary complexes" and "L" means Lipofectamine^{®} 2000 based lipoplexes. The results are given as mean ± SD of three independent experiments in Figs. 7a and 7b.

In the following experiment compound 1 (= C1), compound 3 (= C3), compound 4 (= C4), compound 5 (= C5) and compound 7 (= C7) were compared with respect to their transfection efficiencies. Compound 1 and compound 3 are control compounds of compound 0 (DL). Compound 1 contains a functional NLS targeting sequence and nucleic acid binding sequence but has no hydrophobic moiety to bind the amphiphilic block copolymer. Compound 3 contains a mutated NLS targeting sequence in which the lysine at position 5 has been replaced by threonine. This mutation is known to be transport deficient. Compounds 4, 5 and 7 are compounds containing a functional NLS targeting sequence and a sequence given in italics having high affinity to human airway epithelium cells. The amino acids "GAC...FCG" provide conformational stability for the sequence having high affinity to human airway epithelium cells.

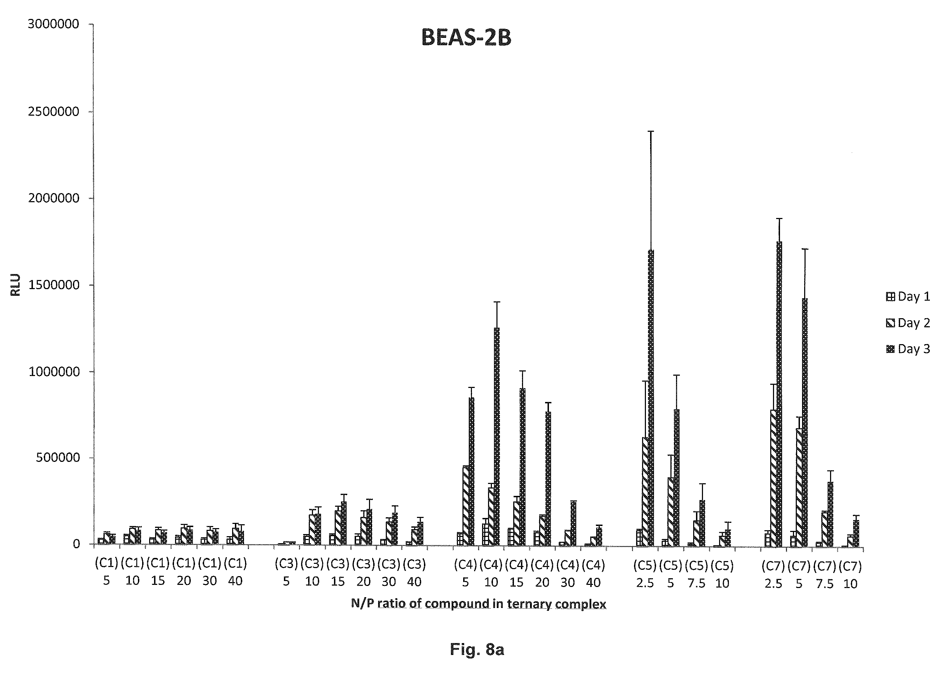

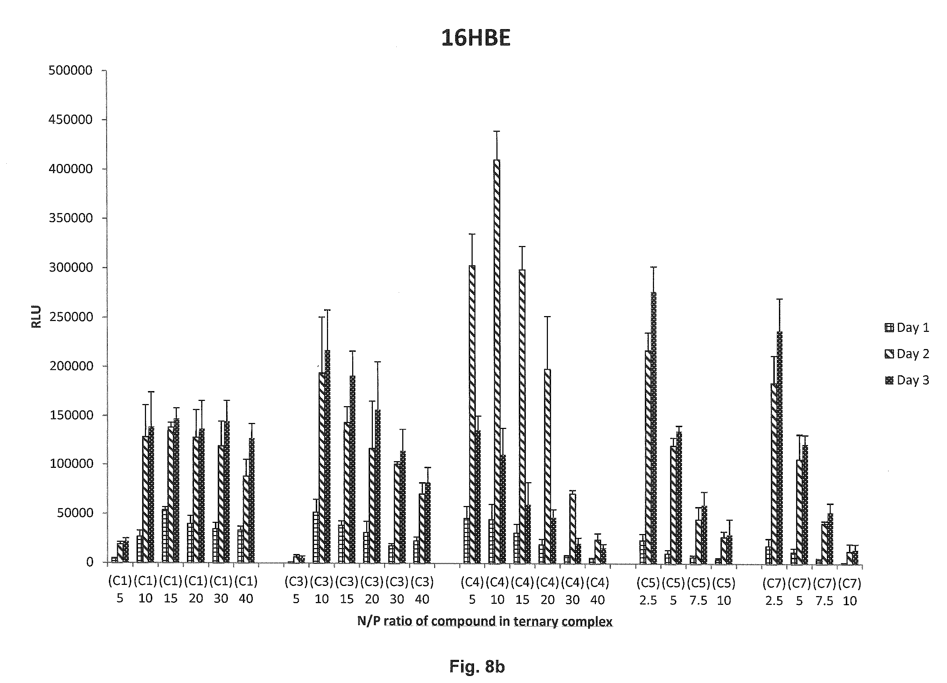

Compounds 5 and 7 have the same sequence having high affinity to human airway epithelium cells but differ in the nucleic acids binding section. Nucleic acid binding section of compound 5 has eight lysine and five arginine residues whereas the corresponding section of compound 7 contains four lysine and eight arginine residues. Transfection efficiencies of ternary complexes containing MetLuc pDNA and different of the above compounds at different N/P ratios (5, 10, 15, 20, 30 and 40) were compared in cells of cell lines BEAS-2B and 16HBE. The results are given in Fig. 8a and 8b. The experiments were performed as follows:
Compound 1 and compound 3 at N/P ratios from 5 to 40 were complexed with P704 at concentrations of 50 (w/w, relative to nucleic acids, for BEAS-2B cell transfection - Fig. 8a) and 63 (w/w, relative to nucleic acids, for 16HBE cell transfection - Fig. 8b) followed by addition of Metluc pDNA (400 ng/well).

Compound 4 at N/P ratios from 5 to 40 was complexed with P704 at concentrations of 50 (w/w, relative to nucleic acids, for BEAS-2B cell transfection - Fig. 8a) and 63 (w/w, relative to nucleic acids, for 16HBE cell transfection - Fig. 8b) followed by addition of Metluc pDNA (400 ng/well). Compound 5 and compound 7 at N/P ratios from 2.5 to 10 were complexed with P704 at concentrations of 50 (w/w, relative to nucleic acids, for BEAS-2B cell transfection - Fig. 8a) and 63 (w/w, relative to nucleic acids, for 16HBE cell transfection - Fig. 8b) to form ternary complexes. The same transfection procedure of previous study was applied. Luciferase activity was expressed in RLU. The data are given as mean ± standard deviation (SD) (n>5).

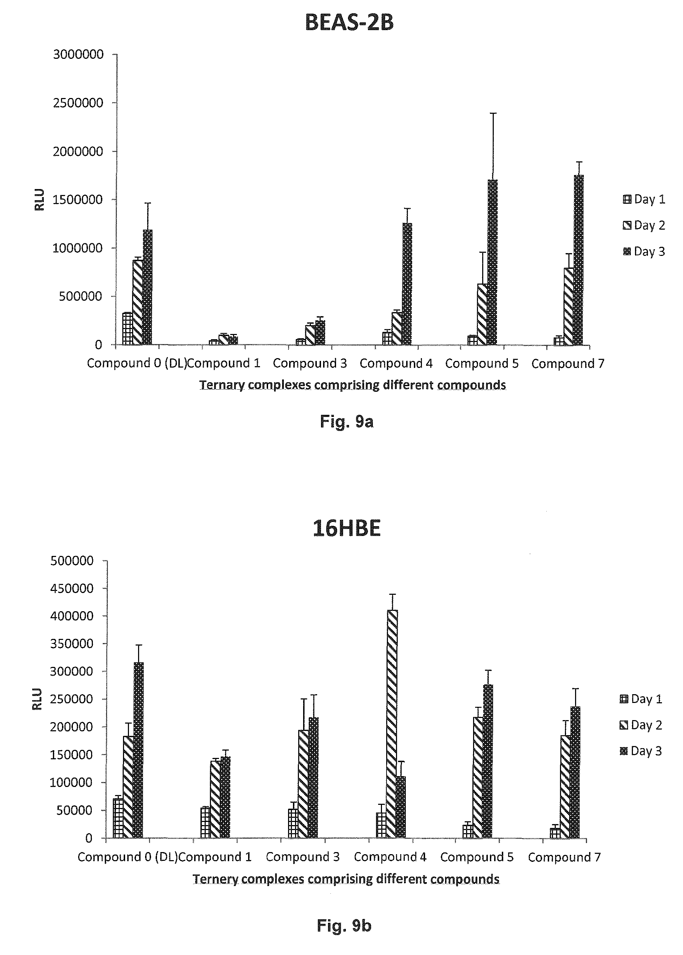

Maximum transfection efficiencies of the ternary complexes was compared with that of compound 0 (DL) containing ternary complexes under the following conditions:
The concentration of P704 in ternary complexes for transfection of BEAS-2B cells was 50 (w/w, relative to nucleic acids). For transfection of 16HBE cells it was 63 (w/w, relative to nucleic acids). The N/P ratios of the different compounds in the ternary complexes were as follows:
   For transfection of BEAS-2B cells: Compound 0 (DL) at 30, compound 1 at 20, compound 3 at 15, compound 4 at 10, compound 5 and compound 7 at 2.5. Results are shown in Fig. 9a.

For transfection of 16HBE cells: Compound 0 (DL) at 20, compound 1 at 15, compound 3 at 10, compound 4 at 10, compound 5 and compound 7 at 2.5. Results are shown in Fig. 9b.

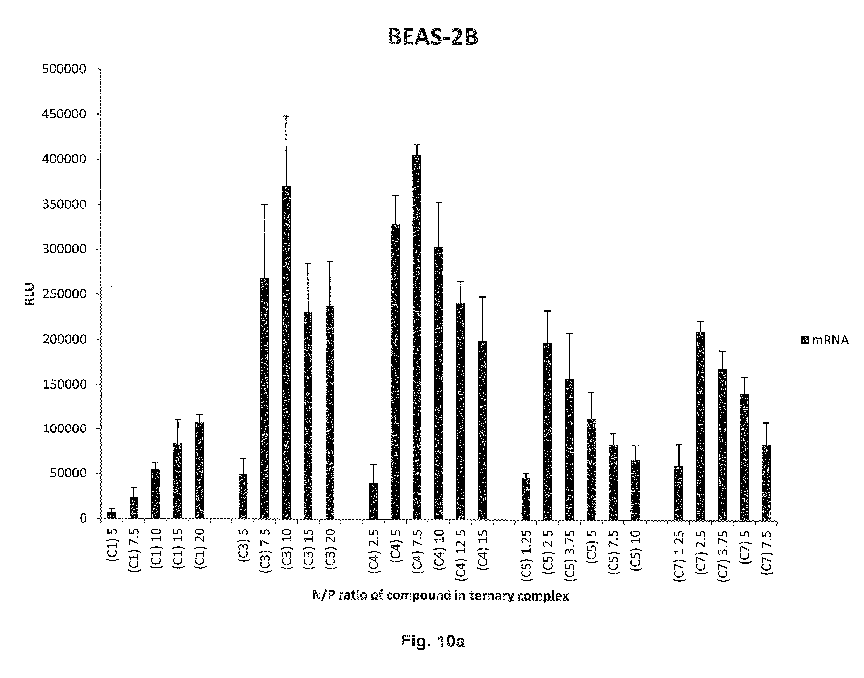

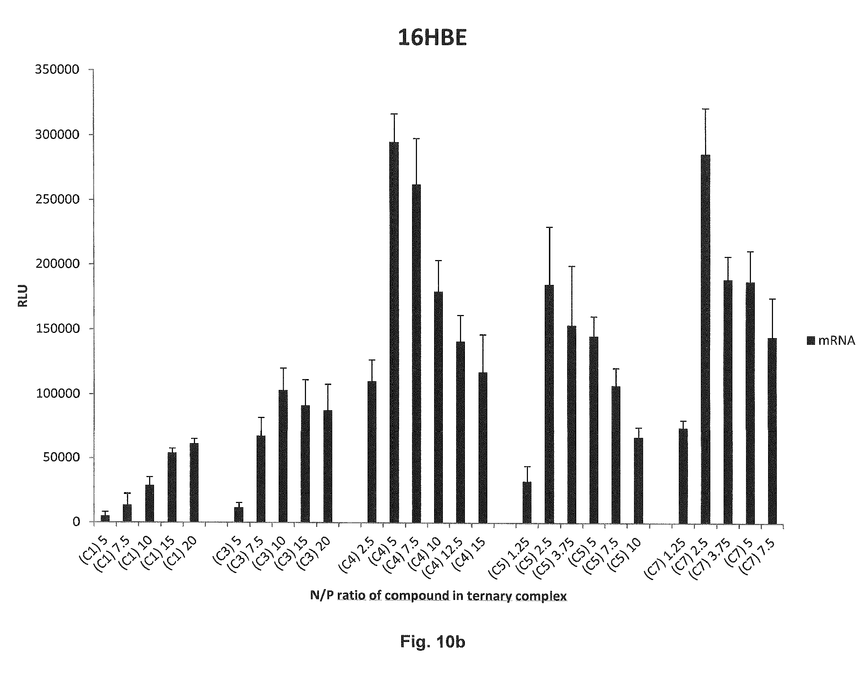

Transfection efficiencies of ternary complexes containing MetLuc mRNA and different synthetic compounds (C1, C3, C4, C5 and C7) at different N/P ratios in cells of cell lines BEAS-2B and 16HBE were compared. The results are given in Figs. 10a and 10b. The experiments were performed as follows:
Compound 1 and compound 3 at N/P ratios from 5 to 20 were complexed with P704 at concentrations of 63 (w/w, relative to nucleic acids) followed by addition of Metluc mRNA (400 ng/well). Compound 4 at N/P ratios from 2.5 to 15 was complexed with P704 at concentrations 63 (w/w, relative to nucleic acids) followed by addition of Metluc mRNA (400 ng/well). Compound 5 and compound 7 at N/P ratios from 2.5 to 10 were complexed with P704 at concentrations of 63 (w/w, relative to nucleic acids) to form ternary complexes. The same transfection procedure of previous study was applied. Luciferase activity was expressed in RLU. The data are given as mean ± standard deviation (SD) (n>5).

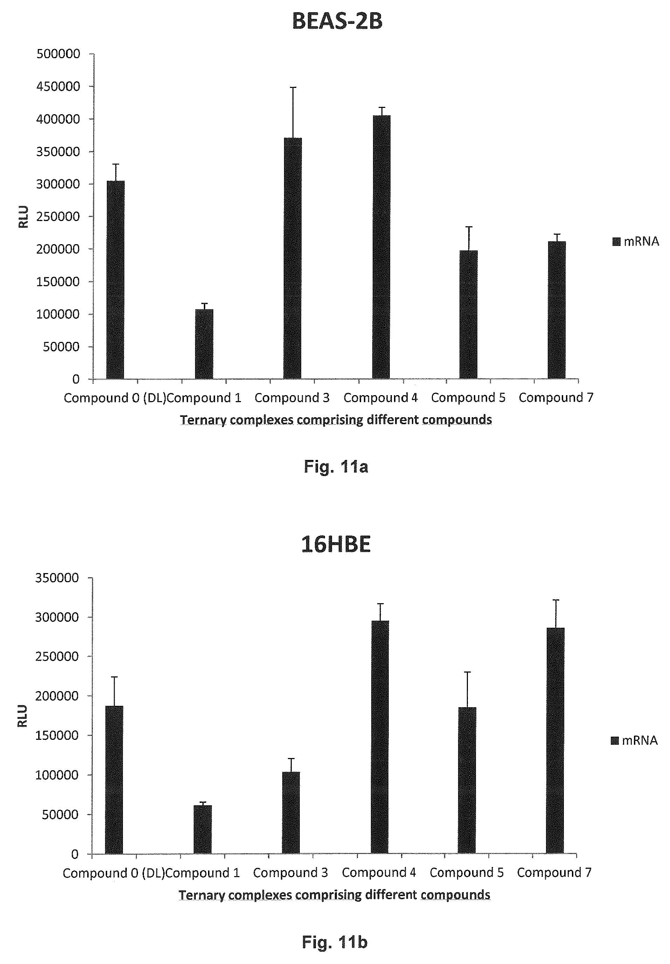

Maximum transfection efficiencies of the ternary complexes was compared with that of compound 0 (DL) containing ternary complexes under the following conditions:
The concentration of P704 in ternary complexes for transfection of both cell lines was 63 (w/w, relative to nucleic acids). The N/P ratios of the different compounds in the ternary complexes were as follows:
   For transfection of BEAS-2B cells: Compound 0 (DL) at 10, compound 1 at 20, compound 3 at 10, compound 4 at 7.5, compound 5 and compound 7 at 2.5. Results are shown in Fig. 11 a.

For transfection of 16HBE cells: Compound 0 (DL) at 10, compound 1 at 20, compound 3 at 10, compound 4 at 5, compound 5 and compound 7 at 2.5. Results are shown in Fig. 11 b.

## Claims

1. Synthetic compound for improving efficiency of transfection of eukaryotic cells by means of an amphiphilic block copolymer which synthetic compound comprises a peptide having a targeting sequence and a nucleic acid binding sequence which nucleic acid binding sequence comprises or consists of at least four consecutive amino acid residues which are positively charged at pH 7.4, **characterized in that** the synthetic compound further comprises a hydrophobic moiety covalently linked to the peptide.

2. Synthetic compound according to claim 1, wherein the targeting sequence comprises or consists of a nuclear localization signal (NLS) sequence and/or a human airway epithelium ligand sequence.

3. Synthetic compound according to claim 1 or 2, wherein the positively charged amino acid residues comprise or consist of histidine residue(s), arginine residue(s) and/or lysine residue(s) and/or positively charged analog(s) of amino acid residue(s).

4. Synthetic compound according to any of the preceding claims, wherein the hydrophobic moiety is covalently linked to the amino terminus or to the carboxy terminus of the peptide.

5. Synthetic compound according to any of the preceding claims, wherein the hydrophobic moiety comprises or consists of lipoic acid, lipoamide, a tetradecyl residue, a cholesteryl residue, or a peptide having a sequence with more than 50% amino acid residues with hydrophobic side chains, in particular amino acid residues of phenylalanine, tyrosine and/or tryptophan.

6. Synthetic compound according to any of the preceding claims, wherein the peptide consists of the targeting sequence and the nucleic acid binding sequence or wherein the peptide comprises or consists of amino acid sequence SEQ ID NO: 1, SEQ ID NO: 2, SEQ ID NO: 4, SEQ ID NO: 5, or SEQ ID NO: 6.

7. Synthetic compound according to any of the preceding claims, wherein the maximal length of the peptide is 30 amino acid residues.

8. Pharmaceutical composition comprising the synthetic compound according to any of the preceding claims together with a pharmaceutically acceptable carrier.

9. Synthetic compound according to any of claims 1 to 7 for use in the treatment of a genetically caused disease of a human being or an animal by gene therapy, wherein the synthetic compound is used together with the amphiphilic block copolymer and a nucleic acid to be transferred in cells of the human being or animal.

10. Synthetic compound according to claim 9 for use in the treatment of a genetically caused disease of a human being or an animal by gene therapy, wherein the amphiphilic block copolymer is a poloxamer, in particular a poloxamine, in particular an ethylene oxide - propylene oxide copolymer with tetraether with (1,2-ethanedinitrilo)tetrakis(propanol), in particular poloxamine 304, 704 or 904.

11. Synthetic compound according to claim 9 or 10 for use in the treatment of a genetically caused disease of a human being or an animal by gene therapy, wherein the compound is present in a nebulized form.

12. Use of the synthetic compound according to any of claims 1 to 7 together with an amphiphilic block copolymer and a nucleic acid to be transferred in eukaryotic cells for an *in vitro* transfection of the eukaryotic cells in cell culture.

13. Use according to claim 12, wherein the amphiphilic block copolymer is a poloxamer, in particular a poloxamine, in particular an ethylene oxide - propylene oxide copolymer with tetraether with (1,2-ethanedinitrilo)tetrakis(propanol), in particular poloxamine 304, 704 or 904.

14. Method for an *in vitro* transfection of eukaryotic cells in cell culture comprising the following steps:
a1) Mixing a plurality of molecules of the synthetic compound according to any of claims 1 to 7 with a plurality of molecules of an amphiphilic block copolymers to allow the formation of binary complexes,
b1) mixing the formed complexes with a plurality of molecules of a nucleic acid to be transferred into the cells to allow the formation of ternary complexes,
or
a2) mixing a plurality of molecules of a nucleic acid to be transferred into the cells with a plurality of molecules of an amphiphilic block copolymers to allow the formation of binary complexes,
b2) mixing the formed complexes with a plurality of molecules of the synthetic compound according to any of claims 1 to 7 to allow the formation of ternary complexes
and
c) contacting the ternary complexes with the cells to be transfected.

15. Method according to claim 14, wherein the amphiphilic block copolymer is a poloxamer, in particular a poloxamine, in particular an ethylene oxide - propylene oxide copolymer with tetraether with (1,2-ethanedinitrilo)tetrakis(propanol), in particular poloxamine 304, 704 or 904.
